# EUROPÄISCHE PATENTANMELDUNG

(11) **EP 2 987 526 A1**
(43) Veröffentlichungstag der Anmeldung: **24.02.2016**
(21) Anmeldenummer: 15167567.5
(22) Anmeldetag: 13.05.2015
(51) Int. Cl.: A61N 1/05

(54) **MEDIZINISCHES IMPLANTAT MIT EINER FIXIERVORRICHTUNG**

(30) Priorität: 21.08.2014 US 201462039931 P
(71) Anmelder: BIOTRONIK SE & Co. KG, 12359 Berlin (DE)
(72) Erfinder: Dörr, Thomas, 12437 Berlin (DE); Diebold, Michael, 12169 Berlin (DE)
(74) Vertreter: Galander, Marcus

(57) **Zusammenfassung**

Die Erfindung betrifft ein Medizinisches Implantat (100) zum Einführen in den menschlichen und/oder tierischen Körper (200). Eine helixförmigen Fixiervorrichtung (130) ist zur Fixierung des Implantats (100) an einem Implantationsort (200) vorgesehen, welche mit einer Sperrvorrichtung (140) an der Fixiervorrichtung (130) und/oder einer Implantatoberfläche (102) gekoppelt ist, die ein selbsttätiges Lösen der Fixiervorrichtung (130) zumindest hemmt.

## Beschreibung

Die Erfindung betrifft ein medizinisches Implantat mit einer Fixiervorrichtung zum Einführen in den menschlichen und/oder tierischen Körper.

Es ist bekannt, medizinische Implantate mittels Fixiervorrichtungen am Implantationsort zu fixieren. Hierzu werden unter anderem helixförmige Fixiervorrichtungen eingesetzt, bei denen eine Helix in das Gewebe eingeschraubt wird, welche das Implantat am Gewebe verankert. Um ein selbstständiges Herausdrehen der Helix zu verhindern, werden diese durch den Chirurgen mittels einer oder mehreren Nähten fixiert. Dies setzt voraus, dass der Implantationsort ausreichend zugänglich ist

Der Erfindung liegt die Aufgabe zugrunde, ein medizinisches Implantat mit einer helixförmigen Fixiervorrichtung zu schaffen, welche auch an schwer zugänglichen Implantationsorten sicher fixiert werden kann.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird ein medizinisches Implantat zum Einführen in den menschlichen und/oder tierischen Körper vorgeschlagen, bei dem eine helixförmigen Fixiervorrichtung zur Fixierung des Implantats an einem Implantationsort vorgesehen ist, welche mit einer Sperrvorrichtung an der Fixiervorrichtung und/oder einer Implantatoberfläche gekoppelt ist, die ein selbsttätiges Lösen der Fixiervorrichtung zumindest hemmt.

Vorteilhaft kann die Dislokation eines nur mit einer Helix fixierten Implantats mittels der Sperrvorrichtung verhindert werden. Dies ermöglicht es, ein Implantat auch an nur schwer zugänglichen Stelle zu positionieren und daher viele Implantationsorte zugänglich zu machen, die bisher ausgeschlossen werden müssen. So ist beispielsweise die Positionierung eines epikardialen Schrittmachers möglich, der tief zwischen Myokard und Perikard geschoben werden kann. Gleichzeitig ist der Platzbedarf der Sperrvorrichtung gering, so dass das positionierte Implantat wenig Platz benötigt.

Gemäß einer günstigen Weiterbildung kann die Sperrvorrichtung ein oder mehrere Widerhaken an der Fixiervorrichtung umfassen. Die Widerhaken können bereits bei der Positionierung der Helix von dieser abstehen, wobei sie so gerichtet sind, dass ein Einschrauben ins Gewebe nicht behindert ist, ein Zurückschrauben jedoch gehemmt ist. Ebenso kann vorgesehen sein, dass Widerhaken erst nach dem Einschrauben der Helix ins Gewebe dringen. Dies ist günstig, wenn eine Repositionierung des Implantats erwünscht ist, wobei die Helix zunächst wieder aus dem Gewebe ausgeschraubt werden soll.

Gemäß einer günstigen Weiterbildung kann die Sperrvorrichtung ein oder mehrere Widerhaken an der Implantatoberfläche umfassen. Die Widerhaken können bereits bei der Positionierung der Helix von dieser abstehen, wobei sie so gerichtet sind, dass ein Einschrauben ins Gewebe nicht behindert ist, ein Zurückschrauben jedoch gehemmt ist. Ebenso kann vorgesehen sein, dass Widerhaken erst nach dem Einschrauben der Helix ins Gewebe dringen. Dies ist günstig, wenn eine Repositionierung des Implantats erwünscht ist, wobei die Helix zunächst wieder aus dem Gewebe ausgeschraubt werden soll.

Denkbar ist auch eine Kombination, bei der Widerhaken sowohl an der Fixiervorrichtung als auch an der Implantatoberfläche vorgesehen sein können.

Gemäß einer günstigen Weiterbildung kann wenigstens ein Widerhaken zeitweise mit einer resorbierbaren Beschichtung abgedeckt sein, welche dazu vorgesehen ist, den wenigstens einen Widerhaken freizugeben, um das Implantat am Implantationsort zu fixieren. Insbesondere kann der Widerhaken eine Federkraft aufweisen, wobei die Beschichtung den Widerhaken gegen seine Federkraft an der Fixiervorrichtung und/oder Implantatoberfläche fixiert. Dies erlaubt eine besonders einfache Repositionierung des Implantats, bei der die Fixiervorrichtung wiederholt in das Gewebe eingeschraubt und wieder daraus entfernt werden und das Implantat verschoben werden oder entfernt werden kann. Dabei können die Widerhaken auch in Form von Lamellen ausgebildet sein.

Gemäß einer günstigen Weiterbildung kann die Fixiervorrichtung und/oder die Implantatoberfläche einen oder mehrere Hinterschnitte aufweisen, wobei der Hinterschnitt einen Hohlraum begrenzt, der zeitweise mit einem resorbierbaren Material gefüllt ist. Dies erlaubt eine besonders einfache Repositionierung des Implantats, bei der die Fixiervorrichtung wiederholt in das Gewebe eingeschraubt und wieder daraus entfernt werden und das Implantat verschoben werden oder entfernt werden kann. Dabei können die Widerhaken auch in Form von Lamellen ausgebildet sein.

Gemäß einer günstigen Weiterbildung kann die Fixiervorrichtung eine äußere und eine innere Helix umfassen, wobei die innere Helix geometrisch gegen die äußere Helix so verstimmt ist, dass eine eindeutige Vorzugsbewegungsrichtung der inneren Helix vorgegeben ist. Die äußere Helix dient dabei als Führungshelix, welche das Einschrauben der inneren Helix in das Gewebe erlaubt, jedoch ein Zurückschrauben der inneren Helix unterbindet. Insbesondere kann die äußere Helix und/oder innere Helix wenigstens bereichsweise eine Gleitbeschichtung aufweisen. Hiermit wird ein Zurückschrauben der äußeren Helix in das Implantatinnere erleichtert.

Gemäß einer günstigen Weiterbildung kann die Sperrvorrichtung ein oder mehrere ausschiebbare Dorne aufweisen, die aus der Implantatoberfläche ausfahrbar sind. Insbesondere kann die Fixiervorrichtung mit einem einen Aktor gekoppelt sein, welcher die Fixiervorrichtung aus der Implantatoberfläche ausfährt. Vorteilhaft kann der Aktor den Dorn oder die Dorne simultan mit der Fixiervorrichtung aus der Implantatoberfläche herausbewegen. Vorteilhaft kann der Aktor gedreht werden, um die Fixiervorrichtung und die Dornen aus der Implantatoberfläche herauszubewegen. Zweckmäßigerweise können die Dorne so ausgelegt sein, dass diese in ihrer Endposition gleichzeitig eine Sperrfunktion gegen eine Drehbewegung des Aktors bewirken.

Gemäß einer günstigen Weiterbildung kann die Fixiervorrichtung und/oder die Implantatoberfläche wenigstens bereichsweise mit einem biokompatiblen Kleber beschichtet sein. Insbesondere kann der Kleber wenigstens einen der Stoffe Fibrinkleber, Cyanoacrylatdispensation aufweisen. Hiermit ist es möglich, das Implantat besonders innig mit dem Gewebe zu verbinden.

Die Erfindung ist nachfolgend beispielhaft, anhand von in Zeichnungen dargestellten Ausführungsbeispielen, näher erläutert. Es zeigen in schematischer Darstellung:
- Fig. 1: in Schnittansicht ein Implantat mit einer mittels Widerhaken fixierten Helix nach einem Ausführungsbeispiel der Erfindung;
- Fig. 2: in Schnittansicht ein Implantat, das mit Widerhaken an einer Gehäuseunterseite des Implantats fixiert ist nach einem Ausführungsbeispiel der Erfindung;
- Fig. 3: eine Ansicht der Gehäuseunterseite des Implantats aus Figur 2;
- Fig. 4: in Schnittansicht ein Detail eines Implantats mit Widerhaken nach einem Ausführungsbeispiel der Erfindung, welche mit einer bioresorbierbaren Oberflächenbeschichtung abgedeckt sind;
- Fig. 5: eine Ansicht eines freigelegten Widerhakens des Implantats aus Figur 4;
- Fig. 6: in Schnittansicht ein Detail eines Implantats mit Widerhaken nach einem Ausführungsbeispiel der Erfindung, welche in eine Oberfläche integriert und teilweise mit bioresorbierbarem Material umgeben sind; und
- Fig. 7: eine Ansicht eines freigelegten Widerhakens des Implantats aus Figur 6.
- Fig. 8: in Schnittansicht ein Implantat mit einer mittels ausschiebbaren Dornen fixierten Helix in eingefahrenem Zustand nach einem Ausführungsbeispiel der Erfindung;
- Fig. 9: in Schnittansicht das mittels ausgeschobenen Dornen fixierte Implantat aus Figur 8;

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Die Erfindung ermöglicht es, ein lediglich mit einer Helix im Körpergewebe befestigtes Implantat 100 vor eine Dislokation zu sichern.

Allgemein beschreibt die Erfindung ein medizinisches Implantat mit einer helixförmigen Fixiervorrichtung für die Fixierung des Implantats in einem Körpergewebe und einer Sperrvorrichtung, die nach endgültiger Positionierung des Implantats vor, während oder nach Abschluss des Einschraubens der Helix in das Körpergewebe eine form- und/oder kraftschlüssige Verbindung mit demselben Körpergewebe derart herstellt, das ein Herausdrehen des Implantates verhindert wird. Dabei können auch verschiedenartige, im folgenden beschriebene Sperrvorrichtungen miteinander in Kombination vorgesehen sein.

Die Erfindung ist am Beispiel eines epikardialen Schrittmachers als Implantat im Folgenden näher erläutert.

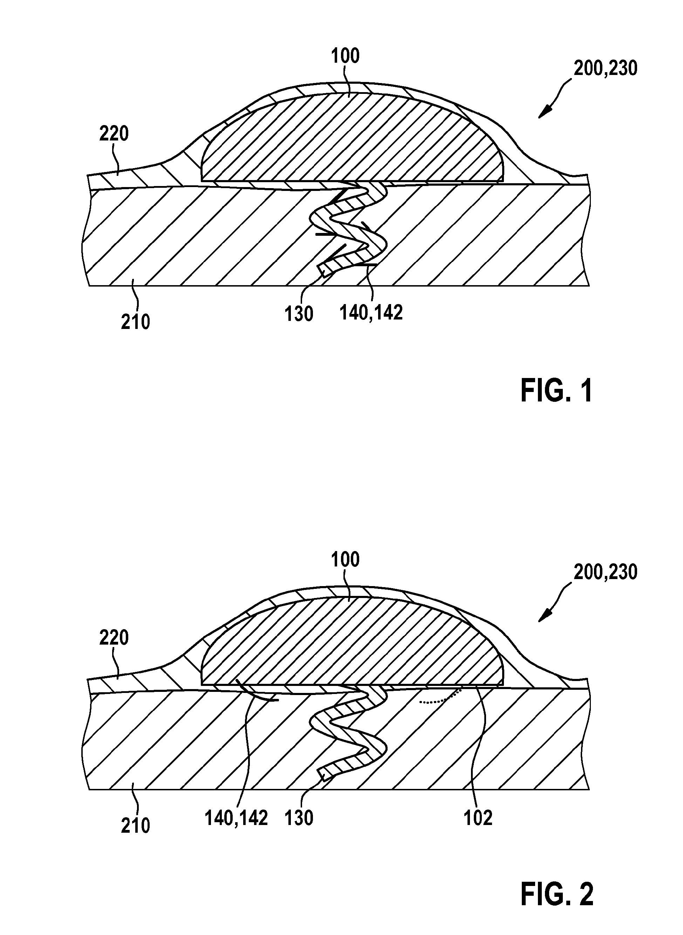

Figur 1 zeigt in Schnittansicht ein Implantat 100 zum Einführen in den menschlichen und/oder tierischen Körper 200 mit einer mittels Widerhaken 142 einer Sperrvorrichtung 140 fixierten und als Helix ausgebildeten Fixiervorrichtung 130 nach einem Ausführungsbeispiel der Erfindung. Das Implantat 100 wird am Implantationsort 230 zwischen Myokard 210 und Perikard 220 geschoben und mittels der Helix 130 im Myokard 210 fixiert. Auf Grund der sehr heftigen Bewegungen des Schrittmachers auf dem Herzmuskel (Myokard) 210 und gegenüber dem Perikard 220 besteht eine hohe Wahrscheinlichkeit, dass die geschraubte Fixiervorrichtung 130 durch diese Bewegungen im Laufe der Zeit wieder herausgeschraubt wird und das Implantat 100 disloziert. Um diese Dislokation zu verhindern, ist die Fixiervorrichtung 130 an ihrer Oberfläche selbst mit zusätzlichen Widerhaken 140 versehen, so dass damit ein Zurückschrauben der Helix 130 wirkungsvoll verhindert wird. Hiermit kann das Implantat 100 einmalig in das Myokard 210 eingeschraubt werden. Ein selbsttätiges Lösen der Fixiervorrichtung 130 ist durch die Widerhaken 142 zuverlässig gehemmt.

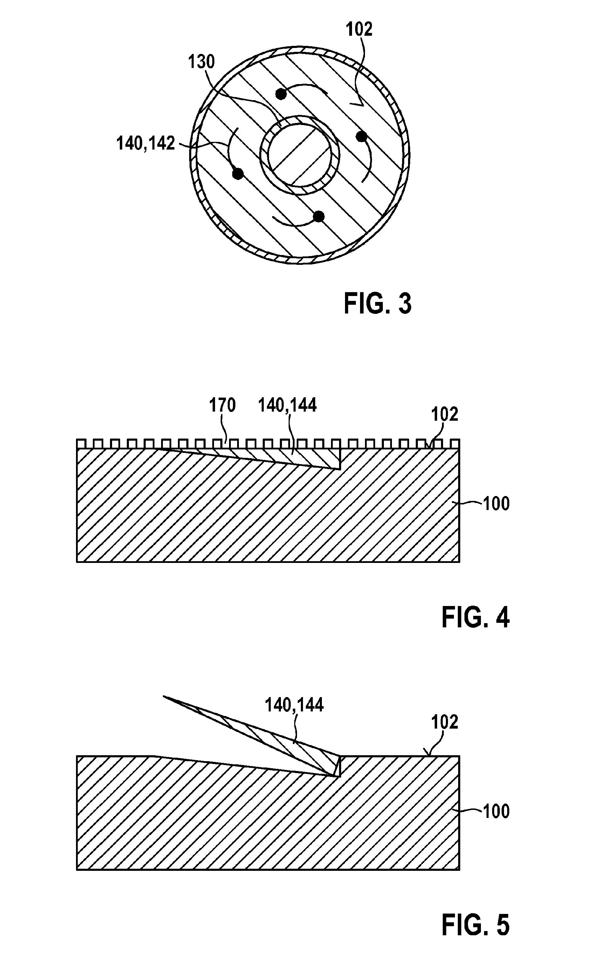

Fig. 2 zeigt in Schnittansicht ein Implantat 100 nach einem Ausführungsbeispiel der Erfindung, das mit Widerhaken 142 an einer Implantatoberfläche 102, beispielsweise einer Gehäuseunterseite des Implantats 100, fixiert ist. Figur 3 zeigt eine Ansicht der Gehäuseunterseite des Implantats 100 aus Figur 2.

Das Implantat 100 wird zwischen Myokard 210 und Perikard 220 geschoben und mittels einer Helix (Fixiervorrichtung 130) im Myokard 210 fixiert. Auf Grund der sehr heftigen Bewegungen des Schrittmachers auf dem Herzmuskel 210 und gegenüber dem Perikard 220 besteht eine hohe Wahrscheinlichkeit, dass die geschraubte Helix (Fixiervorrichtung 130) durch diese Bewegungen im Laufe der Zeit wieder herausgeschraubt wird und Implantat 100 disloziert. Um diese Dislokation zu verhindern, sind an der Unterseite des Implantats 100 an der Implantatoberfläche 102 Widerhaken 140angebracht, die derart angeordnet sind, dass diese eine Drehbewegung entgegen der Einschraubrichtung der Helix verhindern, indem diese Widerhaken 140 sich bei einer derartigen Gegenbewegung in das Myokard 210 hinein aufstellen und diese Gegenbewegung effizient hemmen.

Figur 4 zeigt in Schnittansicht ein Detail eines Implantats 100 mit einem Widerhaken 144 als Sperrvorrichtung 140 nach einem Ausführungsbeispiel der Erfindung, welcher mit einer bioresorbierbaren Beschichtung 170 an einer Implantatoberfläche 102 abgedeckt ist. Derartige Widerhaken 144 können jedoch auch an der Fixiervorrichtung vorgesehen sein (nicht dargestellt). Figur 5 zeigt eine Ansicht eines freigelegten Widerhakens 144 des Implantats 100 aus Figur 4.

Der Widerhaken 140 ist zunächst während des Einführens des Implantats 100 von der bioresorbierbaren Oberflächenbeschichtung 170 abgedeckt, so dass ein Verschieben des Implantats 100 möglich ist. Erst nach Auflösung dieser Beschichtung 170 stellen sich die Widerhaken 144 durch eine Federkraft auf und ragen ins Gewebe, wo sie eine effektive Verdrehsicherung bilden.

Ein geeignetes Material ist beispielsweise Nitinol, Edelstahl, Platin, Tital oder PEEK-Polymere.

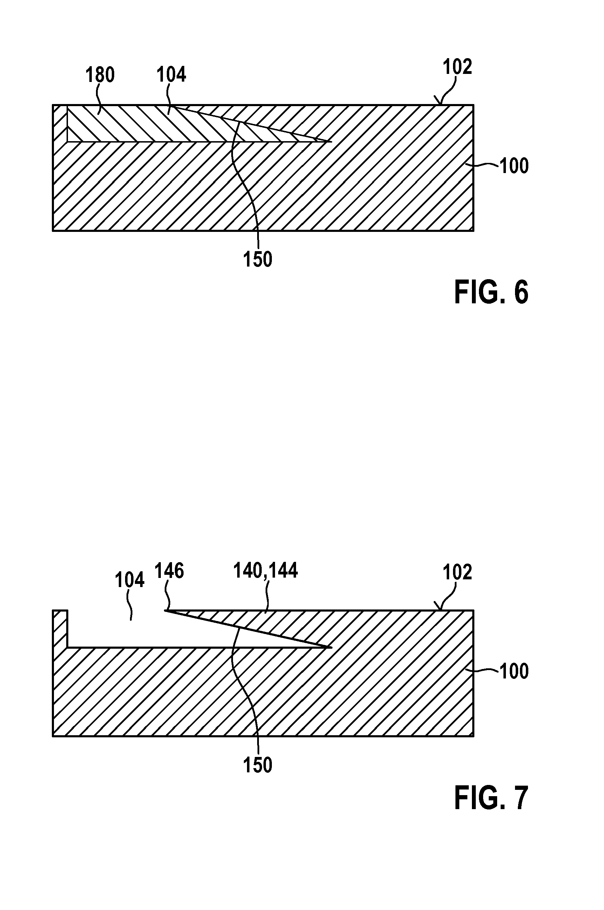

Figur 6 zeigt als Schnittansicht ein Detail eines Implantats 100 nach einem Ausführungsbeispiel der Erfindung mit einem Widerhaken 144 als Sperrvorrichtung 140, welcher als Hinterschnitt 150 in eine Oberfläche integriert ist und teilweise mit bioresorbierbarem Material umgeben ist. Figur 7 zeigt eine Ansicht eines freigelegten Widerhakens 144 des Implantats 100 aus Figur 6.

Ein oder mehrere Hinterschnitte 150 können an der Fixiervorrichtung 130 und/oder die Implantatoberfläche 102 vorgesehen sein. Der Hinterschnitt 170 begrenzt einen Hohlraum 104 an der Oberfläche, der zeitweise mit einem resorbierbaren Material 180 gefüllt ist.

Sobald die Füllung aus resorbierbarem Material 180 aufgelöst ist, kann sich der Widerhaken bei einer unerwünschten Drehbewegung, die zum Lösen der Helix aus dem Gewebe führen würde, mit dem Gewebe verhaken, indem eine scharfe Kante 146 des Widerhakens wirksam wird.

Die Beispiele in den Figuren 4 bis 7 haben den Vorteil, dass die Position des Implantats 100 während der Implantation zunächst noch korrigiert werden kann und erst später die eigentliche Dislokationssicherung wirksam wird. Optional kann hier auch eine Beschichtung 170 bzw. Abdeckung/Füllung 180 benutzt werden, die durch einen externen Trigger, beispielsweise ein gezielter Energieeintrag, den Auflösungsprozess der Beschichtung bzw. Füllung startet. Die Widerhaken 144 können insbesondere an der Implantatoberfläche 102 auch als Lamellenstruktur ausgebildet sein.

In einem zeichnerisch nicht dargestellten Ausführungsbeispiel umfasst die Fixiervorrichtung 130 eine äußere und eine innere Helix, wobei die innere Helix geometrisch gegen die äußere Helix so verstimmt ist, dass eine eindeutige Vorzugsbewegungsrichtung der inneren Helix vorgegeben ist. Die innere Helix kann aus der äußeren Helix in das Gewebe hineingeschraubt werden und das Implantat 100 fixieren, während ein Zurückdrehen der inneren Helix durch deren Formabweichungen, Verwerfungen und dergleichen gehemmt ist. Optional kann die äußere Helix und/oder die innere Helix wenigstens bereichsweise eine Gleitbeschichtung aufweisen, so dass ein Zurückschrauben der äußeren Helix erleichtert wird. Die äußere Helix bildet eine Führungshelix für die innere Helix und hält diese zunächst in einer für das Einschrauben in das Gewebe notwendigen Form.

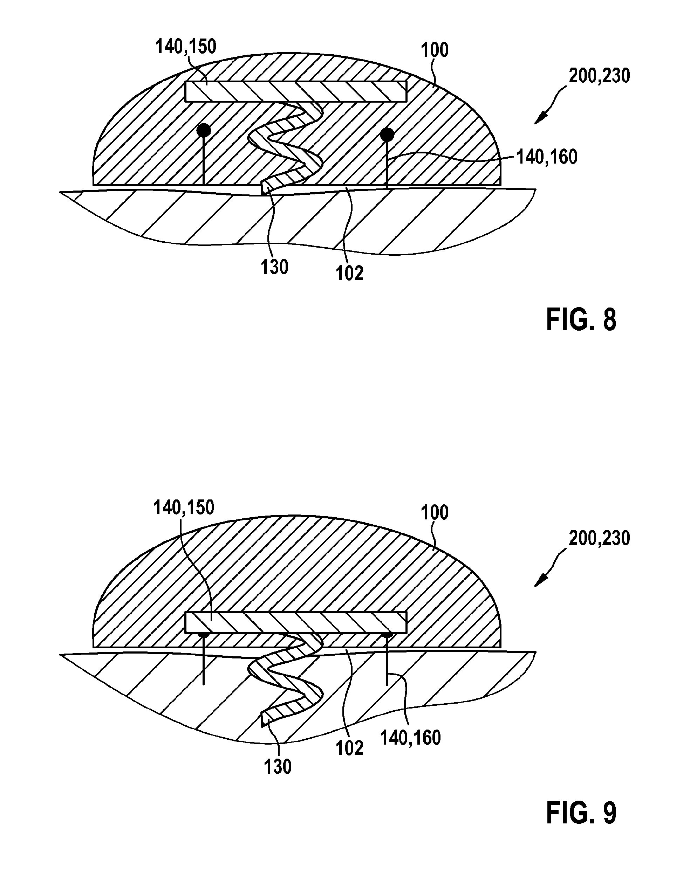

In den Figuren 8 und 9 ist in Schnittansicht ein weiteres Ausführungsbeispiel nach der Erfindung dargestellt, bei dem ein Implantat 100 mit einer mittels ausschiebbaren Dornen 160 einer Sperrvorrichtung 140 fixiert wird. Figur 8 zeigt dabei das Implantat 100 mit einer Helix (Fixiervorrichtung 130) in eingefahrenem Zustand und Figur 9 das mittels ausgeschobenen Dornen 160 fixierte Implantat 100 aus Figur 8.

Das Implantat 100 umfasst eine aus dem Implantatgehäuse ausschraubbare Helix zur Fixierung des Implantats 100 im Gewebe eines Körpers 200. Die Fixiervorrichtung 130 ist hier so ausgebildet, dass diese einen entsprechend dimensionierten Aktor 150 und einen oder mehrere ausfahrbare Dorne 160 umfasst. Der Aktor kann beispielsweise durch eine geeignetes Betätigungsvorrichtung aktiviert werden. Im eingeschraubten Zustand sind die Dorne 160 in das Gehäuse des Implantates 100 zurückgezogen. Wird nun die Helix (Fixiervorrichtung 130) vollständig herausgeschraubt, werden durch den Aktor 150 auch die Dorne 160 derart herausgeschoben, dass diese eine Verdrehsicherung durch Kontakt mit dem Körpergewebe ergeben. Optional kann diese Vorrichtung so ausgelegt werden, dass gleichzeitig eine Sperrfunktion für das Drehen des Aktors 150 durch in den Aktor 150 einrastenden Dorne 160 realisiert wird, wenn die Dorne 160 in ihrer Endposition sind.

Vorteilhaft kann bei allen vorstehend beschriebenen Ausführungsbeispielen die Fixiervorrichtung 130 und/oder die Implantatoberfläche 102 wenigstens bereichsweise mit einem biokompatiblen Kleber beschichtet sein. Der Kleber kann dabei wenigstens einen Fibrinkleber und/oder eine Cyanoacrylatdispensation aufweisen.

Vorteilhaft kann eine dem Kleber zugängliche Beschichtung in Form eines Primers auf der Implantatseite vorgesehen sein. Günstige Materialien als Primer sind z.B. Lösungen der aromatischen Carbonsäuren Benzoesäure und Salicylsäure in Aceton oder Essigsäurebasierte Primer oder alternative biokompatible selbstätzende Primer (Self etching primers).

## Patentansprüche

1. Medizinisches Implantat (100) zum Einführen in den menschlichen und/oder tierischen Körper (200), **dadurch gekennzeichnet, dass** eine helixförmigen Fixiervorrichtung (130) zur Fixierung des Implantats (100) an einem Implantationsort (200) vorgesehen ist, welche mit einer Sperrvorrichtung (140) an der Fixiervorrichtung (130) und/oder einer Implantatoberfläche (102) gekoppelt ist, die ein selbsttätiges Lösen der Fixiervorrichtung (130) zumindest hemmt.

2. Implantat nach Anspruch 1, **dadurch gekennzeichnet, dass** die Sperrvorrichtung (140) ein oder mehrere Widerhaken (142) an der Fixiervorrichtung (130) umfasst.

3. Implantat nach Anspruch 1 oder 2, **dadurch gekennzeichnet, dass** die Sperrvorrichtung (140) ein oder mehrere Widerhaken (142) an der Implantatoberfläche (102) umfasst.

4. Implantat nach einem der vorhergehenden Ansprüche, **dadurch gekennzeichnet, dass** wenigstens ein Widerhaken (144) zeitweise mit einer resorbierbaren Beschichtung (170) abgedeckt ist, welche dazu vorgesehen ist, den wenigstens einen Widerhaken (144) freizugeben, um das Implantat (100) am Implantationsort (230) zu fixieren.

5. Implantat nach Anspruch 4, **dadurch gekennzeichnet, dass** der Widerhaken (144) eine Federkraft aufweist, wobei die Beschichtung (170) den Widerhaken (144) gegen seine Federkraft an der Fixiervorrichtung (130) und/oder Implantatoberfläche (102) fixiert.

6. Implantat nach einem der vorhergehenden Ansprüche, **dadurch gekennzeichnet, dass** die Fixiervorrichtung (130) und/oder die Implantatoberfläche (102) einen oder mehrere Hinterschnitte (150) aufweist, welcher einen oder mehrere Hohlräume (104) begrenzen, der oder die zeitweise mit einem resorbierbaren Material (180) gefüllt sind.

7. Implantat nach einem der vorhergehenden Ansprüche, **dadurch gekennzeichnet, dass** die Fixiervorrichtung (130) eine äußere und eine innere Helix umfasst, wobei die innere Helix geometrisch gegen die äußere Helix verstimmt ist, so dass eine eindeutige Vorzugsbewegungsrichtung der inneren Helix vorgegeben ist.

8. Implantat nach Anspruch 7, **dadurch gekennzeichnet, dass** die äußere und/oder innere Helix wenigstens bereichsweise eine Gleitbeschichtung aufweist.

9. Implantat nach einem der vorhergehenden Ansprüche, **dadurch gekennzeichnet, dass** die Sperrvorrichtung (140) ein oder mehrere ausschiebbare Dorne (160) aufweist, die aus der Implantatoberfläche (102) ausfahrbar sind.

10. Implantat nach Anspruch 9, **dadurch gekennzeichnet, dass** die Fixiervorrichtung (130) mit einem Aktor (150) gekoppelt ist, welcher die Fixiervorrichtung (150) aus der Implantatoberfläche (102) ausfährt.

11. Implantat nach Anspruch 10, **dadurch gekennzeichnet, dass** der Aktor (150) den oder die Dorne (160) simultan mit der Fixiervorrichtung (130) aus der Implantatoberfläche (102) ausfährt.

12. Implantat nach einem der vorhergehenden Ansprüche, **dadurch gekennzeichnet, dass** die Fixiervorrichtung (130) und/oder die Implantatoberfläche (102) wenigstens bereichsweise mit einem biokompatiblen Kleber beschichtet sind.

13. Implantat nach Anspruch 12, **dadurch gekennzeichnet, dass** der Kleber wenigstens einen der Stoffe Fibrinkleber, Cyanoacrylatdispensation aufweist.
